# FASCICULE DE BREVET EUROPEEN

(11) **EP 3 012 311 B1**
(45) Date de publication et mention de la délivrance du brevet: **19.12.2018**
(21) Numéro de dépôt: 15306472.0
(22) Date de dépôt: 22.09.2015
(51) Int. Cl.: C10G 45/60, C07C 5/22, C10G 35/04

(54) **PROCEDE D'ISOMERISATION DE COUPES HYDROCARBONES C5/C6 AVEC RECYCLAGE DES COMPOSES CHLORES**
ISOMERISIERUNGSVERFAHREN VON C5/C6-KOHLENWASSERSTOFFSCHNITTEN MIT RECYCLING DER CHLORVERBINDUNGEN
METHOD FOR ISOMERISATION OF C5/C6 HYDROCARBON FRACTIONS WITH RECYCLING OF CHLORINATED COMPOUNDS

(30) Priorité: 20.10.2014 FR 1460090
(43) Date de publication de la demande: 27.04.2016
(73) Titulaire: AXENS, 92508 Rueil-Malmaison Cedex (FR)
(72) Inventeur: Pigourier, Jérôme, 92190 Meudon (FR); Prevost, Isabelle, 92500 Rueil Malmaison (FR); Watripont, Laurent, 92500 Rueil-Malmaison (FR); Martin, Pierre-Yves, 92500 Rueil Malmaison (FR)
(74) Mandataire: IFP Energies nouvelles

(56) Documents cités:
- US-A- 2 406 778
- US-A- 2 414 371

## Description

### Domaine technique

La présente invention concerne le domaine des procédés d'isomérisation de coupes hydrocarbonées, et plus particulièrement des coupes comprenant des paraffines à 5 et 6 atomes de carbone, et éventuellement pouvant comprendre des paraffines à 4 et/ou 7 voire 8 atomes de carbone.

### Etat de la technique

L'indice d'octane des coupes hydrocarbonées de type C₅ et C₆ issues de la distillation du pétrole brut est généralement compris entre 60 et 75, c'est à dire largement inférieur aux normes en vigueur ; en Europe la spécification sur l'indice d'octane des essences est supérieure ou égale à 95. Le procédé généralement utilisé pour augmenter l'indice d'octane des coupes C₅ et C₆ consiste en une isomérisation des composés normales paraffines (n-paraffines) à faible indice d'octane en paraffines branchées à indice d'octane élevé.

On connait de nombreux procédés d'isomérisation de coupes hydrocarbonées de 5 à 6 atomes de carbone et contenant typiquement une majorité de paraffines. Typiquement, les unités d'isomérisation comprennent des catalyseurs acides permettant la formation de carbocations reconnus comme étant des espèces actives intermédiaires. Les catalyseurs les plus utilisés industriellement comprennent au moins un métal noble, tel que le platine, déposé sur un support, généralement de l'alumine chloré (environ 5 à 10% en masse de chlore). La stabilité et l'activité de ces catalyseurs sont maintenues par l'injection d'agent chlorant, précurseurs d'acide chlorhydrique, afin de maintenir constante la teneur en chlore du catalyseur. Cependant, la présence de chlore sur le catalyseur n'est pas sans inconvénient. En effet, au cours du temps, une élution du chlore, notamment sous forme de chlorure d'hydrogène peut être constatée. Cette élution traduit donc la nécessité de recharger de manière constante le catalyseur en chlore. D'autre part, elle conduit à la présence de chlorure d'hydrogène et/ou d'autres composés chlorés dans les effluents gazeux et liquides issus de l'unité d'isomérisation, ce qui conduit inévitablement à un problème de corrosion des installations, à un problème de formation de dépôts ou de sels à base de chlore, ou bien à un problème de contamination accélérée de catalyseurs pouvant être situés en aval de l'unité d'isomérisation. Il est donc important d'éliminer toutes traces de chlorure d'hydrogène ou de composés chlorés de ces effluents.

Le brevet GB 572,697 décrit la récupération de chlorure d'hydrogène, utilisé comme promoteur catalytique dans les effluents d'unité d'isomérisation de normales paraffines en C₄ ou C₅, par mise en contact avec un solide de type sulfate métallique tel que par exemple le sulfate de cuivre ou de zinc. Les composés chlorés sont ensuite récupérés par élévation de la température.

Il a également été proposé dans les brevets GB 578,085, US 3,188,361 et US 4,275,257 de récupérer les composés chlorés contenus dans les effluents de réacteurs d'isomérisation de paraffines C₄ à C₆ au moyen d'étapes de stripage précédées ou suivies d'étapes d'absorption. Ces procédés utilisent des catalyseurs d'isomérisation solubles comprenant un halogénure métallique, tel que du chlorure d'aluminium ou de zinc, et du chlorure d'hydrogène (HCI), nécessitant de récupérer les quantités significatives de chlorure d'hydrogène introduites dans le réacteur pour les recycler.
Les brevets US 2,414,371 et US 2,406,778 écrivent la récupération des composés chlorés contenus dans les effluents de réacteurs d'isomérisation.

On connait aussi des procédés d'isomérisation utilisant des catalyseurs à base d'alumine chlorée et de platine, dont les produits chlorés sont envoyés dans un épurateur (appelé aussi « scrubber » selon la terminologie anglo-saxonne) pour être neutralisés. Un tel dispositif est notamment décrit dans le document US 4,804,803.

Cependant, les unités de recyclage de composés chlorés de l'état de la technique comportant une absorption du chlore contenu dans le flux dirigé vers l'épurateur par un liquide de lavage valorisable (charge ou produit de l'unité) se font au détriment du rendement global des composés comportant un indice d'octane élevé.

Un but de l'invention est de proposer un nouveau procédé d'isomérisation de paraffines permettant de répondre aux inconvénients précités, tout en permettant d'obtenir un rendement élevé en produits comprenant un indice d'octane élevé.

Le procédé selon l'invention se distingue des procédés selon l'art antérieur en ce qu'il permet d'augmenter le rendement global des composés comportant un indice d'octane élevé, tout en maintenant un taux de recyclage en composés chlorés identique.

### Objets de l'invention

De manière générale, l'invention concerne un procédé d'isomérisation d'une charge de composés hydrocarbonés comportant des composés hydrocarbonés C₅ et/ou C₆, ledit procédé comprenant une boucle de recyclage d'au moins un composé chloré, et dans lequel procédé :
a) on alimente une unité d'isomérisation d'au moins une fraction liquide de ladite charge de composés hydrocarbonés et on effectue l'isomérisation en présence d'un catalyseur chloré ;
b) on alimente une unité de stabilisation comprenant au moins une colonne de stabilisation par l'effluent issu de l'unité d'isomérisation et on effectue une séparation dans ladite unité de stabilisation en :
   ∘ un effluent gazeux comprenant au moins un composé chloré ;
   ∘ un effluent liquide comprenant une concentration C_{A} en composés C₅₊ ; et
   ∘ un flux liquide comprenant au moins un isomérat de la charge de composés hydrocarbonés ;
c) on réalise dans une unité d'absorption comprenant une seule colonne d'absorption :
   - un premier contactage entre l'effluent gazeux issu de l'étape b) et un fluide de lavage, l'alimentation dudit effluent gazeux étant effectuée dans la colonne d'absorption en-dessous de l'alimentation dudit fluide de lavage, ledit fluide de lavage étant choisi parmi une fraction liquide complémentaire de ladite charge et/ou au moins une partie dudit flux liquide comprenant au moins un isomérat de la charge de composés hydrocarbonés issu de l'étape b); ledit fluide de lavage comprenant une concentration C_{B} en composés C₅₊ avec C_{A} < C_{B} ;
   - un second contactage entre le flux gazeux résultant dudit premier contactage et dudit effluent liquide issu de l'étape b); l'alimentation dudit effluent liquide étant effectuée en tête de colonne d'absorption en-dessus dudit fluide de lavage;
d) on extrait de l'unité d'absorption un flux liquide enrichi en composés chlorés et on le renvoie à l'unité d'isomérisation ;
e) on extrait de l'unité de stabilisation ledit flux liquide comprenant au moins un isomérat de la charge de composés hydrocarbonés.

Avantageusement, le flux liquide comprenant au moins un isomérat de la charge de composés hydrocarbonés de l'étape e) est soumis à une séparation dans une unité de séparation de manière à obtenir au moins un flux présentant un indice d'octane plus élevé que ledit flux liquide de l'étape e) et au moins un flux présentant un indice d'octane plus faible que ledit flux liquide de l'étape e).

Plus particulièrement, le flux présentant un indice d'octane plus faible que ledit flux liquide de l'étape e) est au moins en partie recyclé à l'unité d'adsorption en tant que fluide de lavage. Avantageusement, le flux présentant un indice d'octane plus faible que ledit flux liquide de l'étape e) est au moins en partie recyclé en amont de l'unité d'isomérisation, ledit flux recyclé étant mélangé avec la charge de composés hydrocarbonés.

L'unité d'absorption comprend une colonne d'absorption, et on alimente ladite colonne d'absorption lors de l'étape c) :
- dudit fluide de lavage ;
- de l'effluent gazeux issu de l'étape b), l'alimentation dudit effluent gazeux étant effectuée dans la colonne d'absorption en-dessous de l'alimentation dudit fluide de lavage ;
- de l'effluent liquide issu de l'étape b), l'alimentation dudit effluent liquide étant effectuée en tête de colonne d'absorption en-dessus dudit fluide de lavage.

De préférence, ladite charge de composés hydrocarbonés, éventuellement en mélange avec ledit flux liquide comprenant au moins un isomérat de la charge de composés hydrocarbonés issu de l'étape b), est séchée en amont de l'unité d'isomérisation et de l'unité d'absorption. Avantageusement, on refroidit le fluide de lavage en amont de l'unité d'absorption.

Selon l'invention, lorsque le fluide de lavage est une fraction liquide complémentaire de ladite charge, le débit de ladite fraction liquide complémentaire de ladite charge est de préférence compris entre 5 % et 50 % volumique du débit total de ladite charge.

De préférence, la concentration en composés C₄- compris dans l'effluent liquide obtenu à l'étape b) est supérieure à 15% en poids.

Avantageusement, le flux gazeux issu du second contactage dans l'unité d'absorption est extrait et traité dans une unité de traitement des effluents gazeux.

Avantageusement, une partie de l'effluent liquide issu de l'étape b) est envoyée directement à une unité de traitement des effluents gazeux après vaporisation.

Plus précisément, l'étape a) est réalisée à une température comprise entre 100 et 300°C, à une pression comprise entre 0,2 et 3,5 MPa, avec un rapport molaire hydrogène/hydrocarbures compris entre 0,1:1 et 1:1, et avec une vitesse spatiale comprise entre 0,5 et 10 h⁻¹.

De préférence, l'étape a) est réalisée en présence d'un catalyseur chloré comprenant un support en alumine et un métal appartenant au groupe VIII. De préférence, le catalyseur comprend de 2 à 10 % en poids de chlore par rapport au poids total du catalyseur, et de 0,1 à 0,40 % en poids de platine par rapport au poids total du catalyseur.

Selon une variante de réalisation, l'invention concerne un procédé d'isomérisation d'une charge de composés hydrocarbonés comportant des composés hydrocarbonés C₅ et/ou C₆, ledit procédé comprenant une boucle de recyclage d'au moins un composé chloré, et dans lequel procédé :
a') on alimente une unité d'isomérisation d'au moins une fraction liquide de ladite charge de composés hydrocarbonés et on effectue l'isomérisation en présence d'un catalyseur chloré ;
b') on alimente une unité de stabilisation comprenant au moins une colonne de stabilisation par l'effluent issu de l'unité d'isomérisation et on effectue une séparation dans ladite unité de stabilisation en :
   ∘ un effluent gazeux comprenant au moins un composé chloré ;
   ∘ un effluent liquide comprenant une concentration C_{A} en composés C₅₊ ; et
   ∘ un flux liquide comprenant au moins un isomérat de la charge de composés hydrocarbonés ;
c') on réalise dans une unité d'absorption comprenant une seule colonne d'absorption:
   - un premier contactage entre l'effluent gazeux issu de l'étape b') et un fluide de lavage, l'alimentation dudit effluent gazeux étant effectuée dans la colonne d'absorption en-dessous de l'alimentation dudit fluide de lavage, ledit fluide de lavage étant choisi parmi une fraction liquide complémentaire de ladite charge et/ou au moins une partie d'un flux enrichi en composés à faible indice d'octane issue de l'étape e); ledit fluide de lavage comprenant une concentration C_{B} en composés C₅₊ avec C_{A} < C_{B} ;
   - un second contactage entre le flux gazeux résultant dudit premier contactage et dudit effluent liquide issu de l'étape b'); l'alimentation dudit effluent liquide étant effectuée en tête de colonne d'absorption en-dessus dudit fluide de lavage;
d') on extrait de l'unité d'absorption un flux liquide enrichi en composés chlorés et on le renvoie à l'unité d'isomérisation ;
e') on soumet ledit flux liquide comprenant au moins un isomérat de la charge de composés hydrocarbonés de l'étape b') à une séparation dans une unité de séparation de manière à obtenir au moins un flux présentant un indice d'octane plus élevé que ledit flux liquide de l'étape b') et au moins un flux présentant un indice d'octane plus faible que ledit flux liquide de l'étape b').

### Description des figures

Les figures 1a, 1b et 1c représentent schématiquement une installation industrielle d'un procédé d'isomérisation de composés hydrocarbonés connus de l'état de la technique. L'installation comprend une section réactionnelle **1** (appelée aussi ici unité d'isomérisation), une unité de stabilisation **20**, une unité d'absorption **7**, et une unité de séparation **4**.

La figure 2a est une représentation schématique d'une installation industrielle du procédé d'isomérisation selon l'invention.

La figure 2b est une représentation schématique d'une variante de réalisation de procédé non conforme à l'invention.

### Description détaillée de l'invention

### Définitions

Par composé chloré, on entend dans la présente invention tout composé liquide ou gazeux comportant un ou plusieurs atomes de chlore.

Par coupe hydrocarbonée Cₙ, on entend une coupe comprenant des hydrocarbures à n atomes de carbone.

Par coupe Cₙ₊ on entend une coupe comprenant des hydrocarbures à au moins n atomes de carbone.

Par coupe Cₙ₋ on entend une coupe comprenant des hydrocarbures à au plus n atomes de carbone.

Par fluide de lavage, on entend au sens de l'invention, soit une fraction liquide complémentaire de la charge (fraîche) en composés hydrocarbonés, soit au moins une partie d'un flux liquide comprenant au moins un isomérat de la charge de composés hydrocarbonés, de préférence un flux présentant un indice d'octane plus faible que le flux liquide comprenant au moins un isomérat obtenu par séparation dudit fluide comprenant au moins un isomérat, soit encore un mélange de charge et de flux comprenant un isomérat. Par flux recyclé, on entend au moins une partie d'un flux liquide comprenant au moins un isomérat de la charge de composés hydrocarbonés, et de préférence un flux présentant un indice d'octane plus faible que le flux liquide comprenant au moins un isomérat obtenu par séparation dudit flux comprenant au moins un isomérat.

Par composé mono-branché et di-branché on entend respectivement selon l'invention des composés à une ramification, soit un carbone tertiaire, et deux ramifications, comprenant soit deux atomes de carbone tertiaire, soit un atome de carbone quaternaire.

Dans le cadre de l'invention, on définit l'amont et l'aval par rapport au sens d'écoulement de la charge de composés hydrocarbonés, éventuellement en mélange avec un flux recyclé.

### Description détaillée

Les procédés d'isomérisation de coupes comprenant le plus souvent des paraffines à 5 et 6 atomes de carbone, et pouvant comprendre éventuellement des paraffines à 4 et/ou 7 voire 8 atomes de carbone sont connus de l'homme du métier. Les charges utilisées dans le procédé selon l'invention comprennent généralement entre 25 et 100 % en poids de paraffines, plus particulièrement entre 25 et 100 % en poids en normal paraffines, et encore plus particulièrement entre 30 et 60 % en poids de normal paraffines en C₅ et C₆.

Ces procédés utilisent généralement un catalyseur choisi parmi trois type de catalyseurs différents connus : les catalyseurs de type Friedel et Crafts, les catalyseurs zéolithiques bifonctionnels comprenant un métal du groupe VIII déposé sur zéolithe, et les catalyseurs bifonctionnels métal/support à base de métaux du groupe VIII déposés sur alumine, typiquement du platine et contenant généralement un halogène, par exemple du chlore. Le procédé d'isomérisation selon l'invention est de préférence opéré en présence d'un catalyseur à base d'alumine chlorée et de platine. Typiquement, le procédé d'isomérisation est opéré à basse température, par exemple entre 100 et 300°C, de préférence entre 110 et 240 °C, à haute pression, par exemple comprise entre 0,2 et 3,5 MPa, et avec un rapport molaire hydrogène/hydrocarbures compris entre 0,1:1 et 1:1, et avec une vitesse spatiale comprise entre 0,5 et 10 h⁻¹, de préférence comprise entre 1 à 4 h⁻¹.

Les catalyseurs connus utilisés dans les procédés d'isomérisation sont constitués de préférence d'un support en alumine comprenant de préférence de 2 à 10 % en poids de chlore par rapport au poids total du catalyseur, de 0,1 à 0,40 % en poids de platine par rapport au poids total du catalyseur.

Le maintien du taux de chloration du catalyseur nécessite généralement l'appoint en continu d'un composé chloré, tel que le tétrachlorure de carbone, injecté en mélange avec la charge de composés hydrocarbonés, typiquement à une concentration comprise entre 50 et 600 parties par million en poids dans le flux d'entrée du ou des réacteurs de l'unité d'isomérisation qui comprennent le catalyseur.

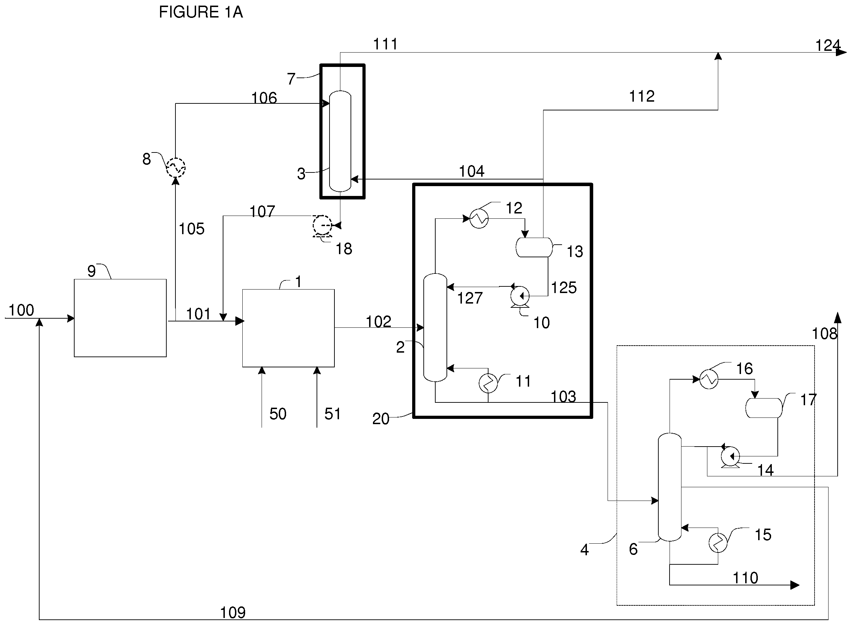

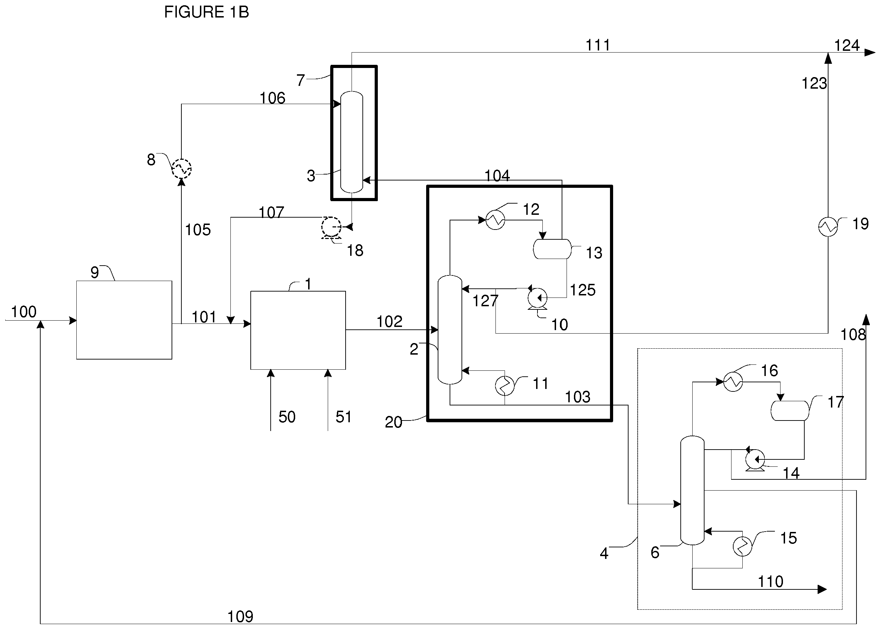

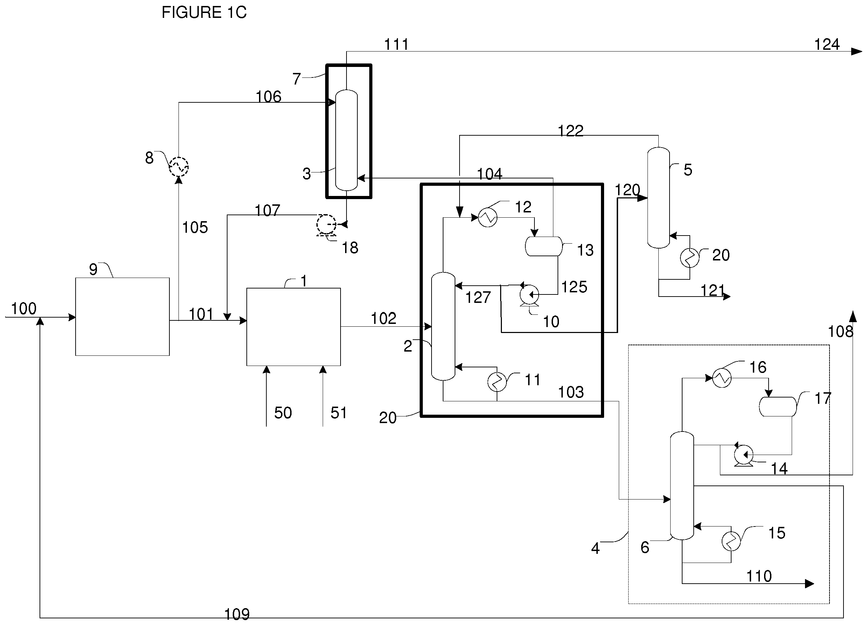

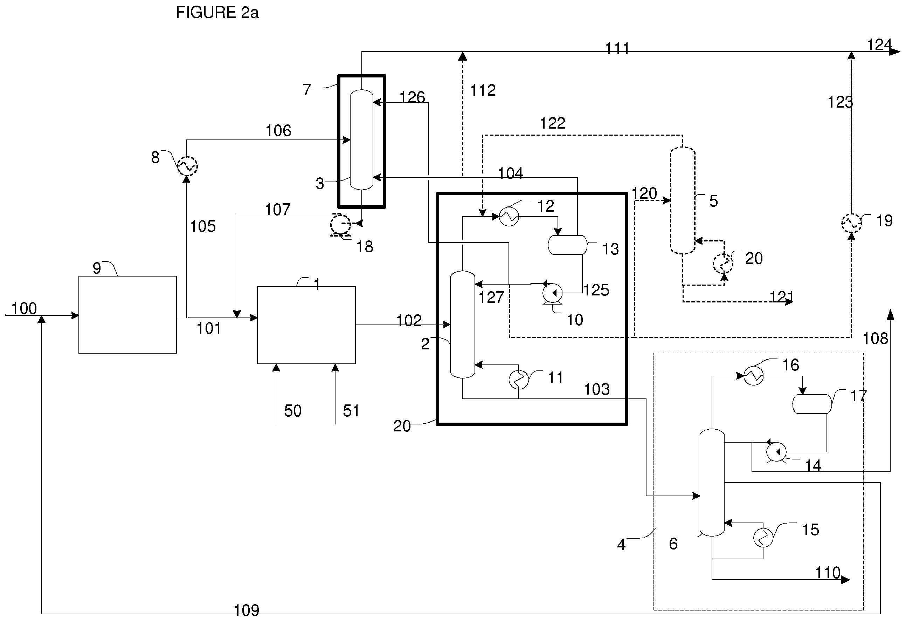

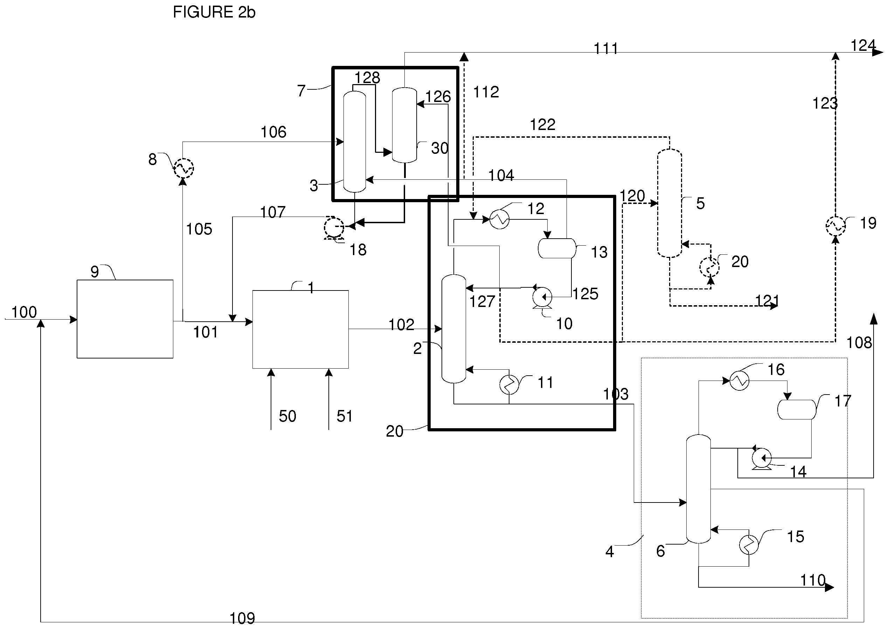

Dans la description qui suit, on décrit dans un premier temps un procédé d'isomérisation selon l'art antérieur (tel que représenté aux figures 1a, 1b et 1c) puis dans un deuxième temps un procédé d'isomérisation conformément à l'invention (tel que représenté en figures 2a et 2b).

En se rapportant aux figures 1a, 1b et 1c, une charge en composés hydrocarbonés, notamment en composés hydrocarbonés de type C₅ et C₆, est dirigée dans le procédé d'isomérisation via une conduite **100**, et est mélangée éventuellement avec des produits de recyclage en provenance de la ligne **109**. Les produits de recyclage seront décrits plus loin dans la description. Le mélange est ensuite séché dans l'enceinte **9**. Le séchage est généralement effectué par adsorption sur tamis moléculaire.

Le flux sec issu du mélange de charge en composés hydrocarbonés comprenant éventuellement des produits de recyclage est ensuite scindé en deux fractions. Une première fraction liquide d'une charge en composés hydrocarbonés est dirigée directement vers la section réactionnelle d'isomérisation **1** via la ligne **101**, et une deuxième fraction (appelé aussi ici fraction liquide complémentaire de la charge en composés hydrocarbonés) est amenée via les lignes **105** et **106** vers une unité d'absorption **7** comprenant une colonne d'absorption **3**. La fraction liquide complémentaire peut être refroidie au moyen d'un échangeur de chaleur **8**. Cette deuxième fraction est dénommée fluide de lavage. Dans ce mode de réalisation, le fluide de lavage est constitué par la fraction liquide complémentaire de la charge en composés hydrocarbonés. On approvisionne de l'hydrogène dans la section réactionnelle **1** via le flux **50** et on approvisionne des composés chlorés dans la section réactionnelle **1** via le flux **51**.

L'effluent issu la section réactionnelle d'isomérisation **1** est envoyé via la ligne **102** vers une unité de stabilisation **20** comprenant une colonne de stabilisation **2**. La colonne de stabilisation **2** permet l'élimination en tête de colonne des composés les plus légers, i.e. le méthane, l'éthane, le propane, le butane, l'élimination de l'excès d'hydrogène, ainsi que de l'acide chlorhydrique et/ou de composés chlorés provenant du catalyseur d'isomérisation. L'effluent gazeux de la tête de colonne de stabilisation **2** est ensuite refroidi au moyen du condenseur **12** et est envoyé dans un ballon de reflux **13**. L'effluent gazeux du ballon de reflux **13** est envoyé, via la ligne **104**, en fond de la colonne d'absorption **3**. L'effluent liquide du ballon de reflux **13** est réinjecté en tête de la colonne de stabilisation, via les lignes **125** et **127**, et au moyen de la pompe **10**.

Au niveau de la colonne d'absorption **3**, la mise en contact de l'effluent gazeux issu de la tête de colonne de stabilisation 2 et du ballon de reflux **13** (via la ligne **104**) par le fluide de lavage (via les lignes **105** et **106**) permet de récupérer une partie de l'acide chlorhydrique et/ou de composés chlorés contenu dans l'effluent gazeux. Cette récupération permet de diminuer la teneur en acide chlorhydrique dans le flux gazeux de tête de la colonne d'absorption **3**, ce dernier étant par la suite envoyé vers une unité de traitement des effluents gazeux, par exemple une unité de neutralisation avec de la soude (non représenté sur la figure 1) via les ligne **111** et **124** (cf. figure 1). Ainsi le coût associé aux traitements des effluents gazeux chargés en acide chlorhydrique est diminué, signifiant par exemple une moindre consommation de soude et par conséquent une moindre production de sels produits lors de la neutralisation.

Le fluide de lavage ayant servi dans la colonne d'absorption **3** est principalement récupéré en fond de colonne et est mélangé, via la ligne **107** et optionnellement au moyen d'une pompe **18**, avec le reste de la charge en composés hydrocarbonés et des éventuels produits de recyclage. L'acide chlorhydrique ainsi entrainé est recyclé vers la section réactionnelle d'isomérisation **1**, ce qui diminue d'autant l'appoint requis en composés chlorés dans ladite unité d'isomérisation.

Cependant, un inconvénient majeur de ce procédé d'isomérisation réside dans le fait que la partie des composés légers, tels que le méthane, l'éthane, le propane et le butane, présents dans l'effluent gazeux de tête de colonne de stabilisation **2** sont aussi ré-entrainés avec le fluide de lavage et sont donc recyclés vers la section réactionnelle de l'unité d'isomérisation **1**, ce qui provoquerait, en l'absence d'autre moyen de les purger, une accumulation inacceptable de ces composés.

Telle qu'illustrée en figure 1a, une solution de l'état de la technique pour répondre à cet inconvénient a été d'envoyer via la ligne **112** directement une partie de l'effluent gazeux de la colonne de stabilisation **2** vers le traitement sans passer par la colonne d'absorption **3**. L'inconvénient est de diminuer le taux de recyclage de l'HCl.

Une autre solution de l'état de la technique pour répondre à cet inconvénient a été de soutirer un distillat liquide du ballon de reflux **13** de la colonne de stabilisation **2**. Ce distillat liquide, dont la quantité dépend de la teneur en produits légers peut être :
- soit dirigé directement vers le traitement des effluents de la colonne d'absorption **3** après vaporisation dans un échangeur **19** (représenté par la ligne **123**) telle que représentée en figure 1b ;
- soit dirigé via la conduite **120** vers une deuxième colonne de stabilisation **5**, telle que représentée en figure 1c, le flux de tête de la colonne de stabilisation **5** est dirigé vers le condenseur **12** de la colonne de stabilisation **2** via la ligne **122**. Le flux de fond de la colonne de stabilisation **5**, essentiellement rebouilli par le rebouilleur **20**, prélevé par la conduite **121** et constitue un produit du procédé. Ce produit comprend essentiellement du gaz de pétrole liquéfié (GPL) valorisable dont la teneur en acide chlorhydrique est généralement compatible avec les spécifications requises, mais peut nécessiter parfois une étape supplémentaire de purification par un procédé de captage du chlore, par exemple par adsorption.

Le fond de la colonne de stabilisation **2**, dont l'acide chlorhydrique a été éliminé, constituant le produit du procédé (isomérat), est récupéré via la ligne **103**, et comprend un ou des produits enrichis en composés à indice d'octane élevé. Ce dernier possède un indice d'octane recherché supérieur à l'indice d'octane de la charge de composés hydrocarbonés en amont de l'unité d'isomérisation. Il peut constituer une essence à haut indice d'octane. Une fraction du flux de fond de la colonne de stabilisation **2** est réchauffée via le rebouilleur **11** et est renvoyée en fond de ladite colonne de stabilisation **2**.

Ce produit peut aussi faire l'objet d'une étape supplémentaire de séparation **4** dans lequel on récupère d'une part un ou plusieurs fluides enrichis en composés à faible indice d'octane qui seront recyclés à la section réactionnelle via la conduite **109**, et d'autre part un ou des produits enrichis en composés à indice d'octane élevé issus des lignes **108** et **110**. Pour ce faire, au moins une partie du flux de fond de la colonne de stabilisation **2** est envoyée dans une unité de séparation **4**, par exemple une colonne de distillation **6**, via la ligne **103**. Ledit flux peut être séparé également par tout autre moyen, par exemple par une colonne de stabilisation, ou encore une colonne d'adsorption. Le flux de tête de colonne **6** est refroidi via le condenseur **16**, est envoyé dans le ballon de reflux **17**. Une partie de l'effluent liquide issu du ballon de reflux **17** est réinjecté en tête de colonne de distillation **6** comme reflux via une pompe **14**, la partie complémentaire de l'effluent liquide issu du ballon de reflux **17** est récupéré via la pompe **14** et la ligne **108**, et comprend des produits enrichis en composés à indice d'octane élevé. On récupère en fond de colonne de distillation **6** via la ligne **110** un flux enrichi en composés d'indice d'octane élevée et en C6+ naphténiques réfractaires ainsi purgés afin d'éviter leur accumulation. Une partie du flux de fond est réchauffé dans un rebouilleur **15** et est renvoyée en fond de ladite colonne de distillation **6**.

Avantageusement, le fluide de lavage envoyé vers la colonne d'absorption **3** via les lignes **105** et **106** peut être préalablement refroidi au moyen d'un échangeur **8** afin d'améliorer le taux de récupération de l'acide chlorhydrique. De préférence, le lavage est réalisé à basse température pour améliorer l'efficacité du lavage, et est plus particulièrement réalisé à température ambiante.

Cependant lors du lavage de l'effluent gazeux de tête de colonne de stabilisation **2** dans les procédés selon l'état de la technique, il se produit inévitablement une perte de composés à cinq atomes de carbone ou plus (C₅₊), alors que ces derniers sont des composés hautement valorisables, ce qui conduit incontestablement à une diminution globale du rendement du procédé d'isomérisation. En effet, dans les procédés selon l'état de la technique, le contactage entre l'effluent gazeux du ballon de reflux **13** et la fraction liquide complémentaire de la charge en composés hydrocarbonés envoyée elle aussi dans unité d'absorption 7 via la ligne **106**, forme un flux gazeux comprenant des composés C₅₊. Les composés C₅₊ compris dans le flux gazeux issu de ce contactage sont alors entrainés vers l'unité de traitement des effluents gazeux, via les ligne **111** et **124**, et ne sont donc pas récupérables. Ainsi, en contrepartie de la récupération des composés chlorés en provenance de l'unité d'isomérisation **2** par l'intermédiaire de la ligne **104**, des composés C₅₊ issus de la fraction liquide de la charge en composés hydrocarbonés sont entrainés, ce qui diminue le rendement global du procédé d'isomérisation de composés hydrocarbonés.

Le procédé selon l'invention permet d'éviter le recyclage en boucle de produits non souhaités, tels que les composés les plus légers, tout en optimisant le recyclage de l'acide chlorhydrique et/ou de composés chlorés, et tout en évitant une perte de composés hydrocarbonés valorisables en tant que produits, et plus particulièrement les composés C₅₊.

Selon l'invention, pour éviter une perte de rendement en composés hydrocarbonés valorisables, i.e. les composés C₅₊, le flux gazeux issu du contactage entre l'effluent gazeux du ballon de reflux **13** envoyé, via la ligne **104**, dans l'unité d'absorption **7** et la fraction liquide complémentaire de la charge en composés hydrocarbonés envoyée dans l'unité d'absorption **7** via la ligne **106**, est contacté avec l'effluent liquide du ballon de reflux **13** injecté dans l'unité d'absorption **7** via la ligne **126**. En effet, l'effluent liquide du ballon de reflux **13** injecté dans l'unité d'absorption **7** via la ligne **126** comprend une teneur en composés C₅₊ moins importante que la concentration en composés C₅₊ de la fraction complémentaire de la charge de composés hydrocarbonés. Ainsi, les composés C₅₊ contenus dans l'effluent gazeux issu du premier contactage se condensent au contact de l'effluent liquide du ballon de reflux **13** injecté dans l'unité d'absorption **7** via la ligne **126**. Plus généralement, pour augmenter le rendement en composés C₅₊ du procédé d'isomérisation selon l'invention, la concentration « C_{A} » en composés C₅₊ de l'effluent liquide issu de l'unité de stabilisation **20**, envoyé dans l'unité d'absorption **7**, doit être inférieure à la concentration « C_{B} » en composés C₅₊ de la fraction liquide complémentaire de la charge en composés hydrocarbonés envoyée dans l'unité d'absorption **7**.

Avantageusement, la concentration en composés C₄₋ présents dans l'effluent liquide issu de l'unité de stabilisation **20**, injecté dans l'unité d'absorption **7**, est supérieure à 15 % en poids, et encore plus préférentiellement supérieure à 30 % en poids.

En se reportant aux figures 2a et 2b (sur ces figures, des références identiques à celles des figures 1a à 1c désignent des éléments identiques) la figure 2a présente un mode de réalisation selon l'invention, une charge en composés hydrocarbonés, notamment en composés hydrocarbonés de type C₅ et C₆, est dirigée dans le procédé d'isomérisation via une conduite **100**, et est mélangée éventuellement avec un flux recyclé comprenant des produits de recyclage en provenance de la ligne **109**.

L'étape de séchage de la charge fraîche peut être réalisée en amont ou en aval du mélange entre ladite charge et les produits recyclés issus de la ligne flux **109**. Dans un mode de réalisation préféré, le séchage est réalisé en aval dudit mélange de manière à éliminer toute présence d'eau susceptible d'être amenée par la ligne **109** de produits recyclés.

Le flux sec issu du mélange de la charge en composés hydrocarbonés, éventuellement en mélange avec le flux recyclé, est ensuite scindé en deux fractions. Une première fraction est dirigée directement vers la section réactionnelle d'isomérisation via la ligne **101**, et une deuxième fraction (fluide de lavage) est amenée via les lignes **105** et **106** dans une unité d'absorption **7**. Dans le cadre du procédé selon l'invention, le débit de fluide de lavage est compris entre 5 % et 50 % volumique du débit total de la charge en composés hydrocarbonés, éventuellement en mélange avec un flux recyclé, de préférence entre 10 % et 30 % volumique.

Dans un mode de réalisation particulier du procédé selon l'invention (non représenté sur les figures), il est possible de scinder la charge de composés hydrocarbonés avant le mélange avec le flux recyclé issu de la ligne **109** et d'envoyer uniquement une fraction de la charge en composés hydrocarbonés vers l'unité d'absorption **7**, en l'absence de flux recyclé.

Dans un autre mode de réalisation particulier du procédé selon l'invention (non représenté sur les figures), il est possible d'envoyer uniquement une fraction de flux recyclé issu de la ligne **109** vers l'unité d'absorption **7**, en l'absence de charge en composés hydrocarbonés. Dans ce mode de réalisation, le fluide de lavage est le flux recyclé, i.e. le flux présentant un indice d'octane plus faible que le flux liquide comprenant au moins un isomérat de la charge de composés hydrocarbonés.

Dans encore un autre mode de réalisation particulier du procédé selon l'invention (non représenté sur les figures), le fluide de lavage est constitué par un mélange d'un fluide comprenant la fraction liquide complémentaire de ladite charge en composés hydrocarbonés et du flux recyclé.

Tel qu'illustré en figure 2a, l'effluent de la section réactionnelle d'isomérisation **1** est envoyé via la ligne **102** vers la colonne de stabilisation **2**, permettant l'élimination en tête de colonne des composés les plus légers, i.e. le méthane, l'éthane, le propane, le butane, l'élimination d'excès d'hydrogène, ainsi que l'acide chlorhydrique et/ou de composés chlorés provenant du catalyseur. L'effluent gazeux de la tête de colonne de stabilisation **2** est ensuite refroidi au moyen du condenseur **12** et est envoyé dans un ballon de reflux **13**. L'effluent gazeux du ballon de reflux **13** est envoyé, via la ligne **104**, en fond de l'unité d'absorption **7** comprenant une colonne d'absorption **3**. Une partie de l'effluent liquide du ballon de reflux **13** est réinjectée comme reflux en tête de la colonne de stabilisation **2**, via les lignes **125** et **127**, éventuellement au moyen de la pompe **10**.

Au niveau de la colonne d'absorption **3**, la mise en contact de l'effluent gazeux issu de la tête de colonne de stabilisation **2** (via la ligne **104**) par le fluide de lavage (via les lignes **105** et **106**) permet de récupérer une partie de l'acide chlorhydrique et/ou de composés chlorés contenu dans l'effluent gazeux. Cette récupération permet de diminuer la teneur en acide chlorhydrique dans l'effluent de tête de la colonne d'absorption **3**, ce dernier étant par la suite envoyé vers une unité de traitement des effluents gazeux (non représenté sur la figure 1) via la ligne **111**.

On définit par « taux de récupération de l'acide chlorhydrique », le rapport entre le débit d'acide chlorhydrique du flux de fond de la colonne d'absorption **3** via la ligne **107** par la somme du débit d'acide chlorhydrique du flux de fond de la colonne d'absorption **3** via la ligne **107** et du débit d'acide chlorhydrique dans l'effluent gazeux de la tête de colonne de stabilisation **3** via la ligne **111**. Le taux de récupération de l'acide chlorhydrique est généralement compris entre 20% et 90%. Ce débit varie en fonction du débit et de la température du fluide de lavage.

Avantageusement, le fluide de lavage envoyé vers la colonne d'absorption **3** via les lignes **105** et **106** peut être préalablement refroidi au moyen d'un échangeur **8** afin d'améliorer le taux de récupération de l'acide chlorhydrique. De préférence, le lavage est réalisé à basse température pour améliorer l'efficacité du lavage, et est plus particulièrement réalisé à température ambiante.

Le fluide de lavage ayant servi dans la colonne d'absorption **3** est essentiellement récupéré en fond de colonne d'absorption **3**, et est mélangé, via la ligne **107**, avec la charge en composés hydrocarbonés en amont de l'unité d'isomérisation, éventuellement en mélange avec un flux recyclé. L'acide chlorhydrique ainsi entrainé est recyclé à la section réactionnelle d'isomérisation et diminue d'autant l'appoint requis en composé chloré.

Dans le mode de réalisation illustré en figure 2a, l'unité d'absorption **7** comprend une seule colonne d'absorption **3**. L'effluent liquide issu du ballon de reflux **13** de la colonne de stabilisation **2** est injectée dans la colonne d'absorption **3** via les lignes **125** et **126**. Cette injection est réalisée dans la section de la colonne d'absorption **3** située au-dessus de l'alimentation en fluide de lavage. Elle permet de diminuer les pertes en composés hydrocarbonés, notamment en C₅₊, issus du premier contactage entre l'effluent gazeux issu du ballon **13** et la fraction liquide complémentaire de la charge en composés hydrocarbonés, et donc ne pouvant plus être récupéré par la suite car le flux de tête de colonne d'absorption **3** est par la suite conduit vers une unité de traitement des effluents gazeux (non représentée sur la figure) via la ligne **111**.

Dans un mode de réalisation alternatif (figure 2b), l'unité d'absorption **7** comprend une première colonne d'absorption **3** et une seconde colonne d'absorption **30**. L'effluent gazeux du ballon de reflux **13** est envoyé, via la ligne **104**, en fond de la colonne d'absorption **3**. La fraction liquide complémentaire de la charge hydrocarbonée est envoyée dans la colonne d'absorption **3** via la ligne **106**. L'alimentation de la fraction liquide complémentaire de la charge hydrocarbonée est située au-dessus de l'alimentation de l'effluent gazeux du ballon de reflux **13**. Le flux gazeux issu du contactage entre la fraction liquide complémentaire de la charge hydrocarbonée et de l'effluent gazeux issu du ballon de reflux **13** est récupéré en tête de la colonne d'absorption **3** puis est envoyé dans une deuxième colonne d'absorption **30** via la ligne **128**. L'effluent liquide issu du ballon de reflux **13** est envoyé via les lignes **125** et **126** dans la colonne d'absorption **30**. L'alimentation de l'effluent liquide issu du ballon de reflux **13** est située au-dessus de l'alimentation de l'effluent gazeux issu du premier contactage dans la colonne d'absorption **3**. Ainsi, on réalise dans la deuxième colonne d'absorption **30** un deuxième contactage entre le flux gazeux issu du premier contactage et l'effluent liquide issu du ballon de reflux **13**. L'effluent gazeux issu du deuxième contactage est récupéré en tête de colonne d'absorption **30** est envoyé vers l'unité de traitement des effluents gazeux via les lignes **111** et **124**. Le fluide de lavage ayant servi dans la colonne d'absorption **3** est essentiellement récupéré en fond des colonnes d'absorption **3** et **30**.

Selon un aspect essentiel du procédé selon l'invention, c'est en effet le second contactage entre l'effluent gazeux résultant du premier contactage et l'effluent liquide, par exemple issu du ballon de reflux **13**, qui permet d'augmenter le rendement global en C₅₊ et de libérer plus de composés C₄₋ dans le flux de la ligne **111**.

Dans un autre mode de réalisation alternatif (non représenté sur les figures), l'unité de stabilisation **20** comprend une colonne de stabilisation **2** alimentée par l'effluent issu de l'unité d'isomérisation **1**, et dans laquelle unité de stabilisation **20** :
- on récupère en fond de colonne de stabilisation **2**, un flux comprenant l'isomérat de la charge de composés hydrocarbonés ;
- on récupère en tête de colonne de stabilisation **2**, un effluent gazeux comprenant au moins en partie des composés légers et des composés chlorés ; et on envoie l'effluent gazeux vers une unité d'absorption **7**
- on récupère par soutirage latéral de la colonne de stabilisation, un effluent liquide comprenant des composés légers et des composés C₅₊, et on envoie l'effluent liquide vers une unité d'absorption **7**. La concentration en composés C₅₊ de l'effluent liquide soutiré latéralement est inférieure à la concentration en C₅₊ compris dans la fluide de lavage.

Comme pour les modes de réalisation précédents, dans l'unité de stabilisation **7**, l'effluent gazeux issu de l'unité de stabilisation **20** est mis en contact avec la fraction liquide complémentaire de la charge de composés hydrocarbonés. Le flux de vapeur résultant de ce premier contactage est par la suite contacté avec l'effluent liquide issu de l'unité de stabilisation **20** afin de récupérer les composés C₅₊ compris dans le flux gazeux résultant du premier contactage.

Le fond de la colonne de stabilisation **2**, dont l'acide chlorhydrique a été éliminé, constituant le produit du procédé (isomérat), est récupéré via la ligne **103**, et comprend un ou des produits enrichis en composés à indice d'octane élevé. Ce dernier possède un indice d'octane recherché supérieur à l'indice d'octane de la charge de composés hydrocarbonés en amont de l'unité d'isomérisation. Il peut constituer une essence à haut indice d'octane. Une fraction du flux de fond de la colonne de stabilisation **2** est réchauffé via le rebouilleur **11** et est renvoyée en fond de ladite colonne de stabilisation **2**.

Dans un mode de réalisation préféré, les produits issus du flux de fond de la colonne de stabilisation **2** sont dirigés vers une unité de séparation **4**, et plus particulièrement, par exemple par distillation au moyen d'une colonne de distillation **6**, ou encore par adsorption. De préférence, la colonne de distillation **6** est un déisohexaniseur. Tel que mieux illustré en figure 2a ou 2b, le déisohexaniseur est une colonne de distillation de laquelle sont soutirés au moins trois flux, à savoir en tête, via la ligne **108**, en soutirage latéral, via la ligne **109**, et en fond, via la ligne **110**.

On extrait de la colonne un flux comprenant des produits présentant un indice d'octane plus faible que le flux liquide issu de la colonne de stabilisation **2** (i.e. le flux de fond de la colonne de stabilisation **2**) par soutirage latéral de la colonne de distillation **6**. Ces produits sont ensuite de préférence recyclés et mélangés avec la charge fraîche vers la section réactionnelle d'isomérisation **1** via la ligne **109**. Le flux présentant un indice d'octane plus élevé que ledit flux liquide issu de la colonne de stabilisation **2** (via la ligne **103**) est extrait en de tête de colonne, via la ligne **108**, et en fond de colonne, via la ligne **110**.

Pour ce faire, au moins une partie du flux de fond de la colonne de stabilisation **2** est envoyée dans une unité de séparation **4**, par exemple une colonne de distillation **6**, via la ligne **103**. Le flux de tête de colonne **6** est refroidi via le condenseur **16**, est envoyé dans le ballon de reflux **17**. Une partie de l'effluent liquide issu du ballon de reflux **17** est réinjecté en tête de colonne de distillation **6** comme reflux via une pompe **14**, la partie complémentaire de l'effluent liquide issu du ballon de reflux **17** est récupéré via la pompe **14** et la ligne **108**, et comprend des produits enrichis en composés à indice d'octane élevé. On récupère en fond de colonne de distillation **6** via la ligne **110** un flux enrichi en composés indice d'octane élevé et en C₆₊ naphténiques réfractaires afin d'éviter leur accumulation. Une partie du flux de fond est réchauffé dans un rebouilleur **15** et est renvoyée en fond de ladite colonne de distillation **6**.

Les produits issus du flux de tête de la colonne de distillation **6** comprend essentiellement des composés C₅ et C₆ di-branchés. Les produits issus du flux de fond de la colonne de distillation **6** comprennent essentiellement des composés paraffinés C₇, du cyclohexane et des naphtènes.

### Exemple

Dans l'exemple qui suit, une comparaison est effectuée entre le procédé d'isomérisation de l'état de la technique, tel qu'illustré en figure 1b (et appelé ici procédé 1), avec le procédé d'isomérisation de l'invention, tel qu'illustré en figure 2a (et appelé ici procédé 2), i.e. un procédé comprenant une seule colonne d'absorption **3** dans l'unité d'absorption **7**. Pour les deux procédés, on réalise une isomérisation d'une charge fraîche en composés hydrocarbonés dont la composition est détaillée en % pds dans le tableau 1 ci-après.

**Tableau 1 : Composition de la charge fraîche et débit massique total de la charge fraîche d'entrée**

| Débit massique total | kg/h | 37 249 |
|---|---|---|
| Isobutane | % poids | 0% |
| n-butane | % poids | 0% |
| Isopentane | % poids | 3% |
| n-pentane | % poids | 27% |
| 2,2-diméthylbutane | % poids | 1% |
| 2,3-diméthylbutane | % poids | 3% |
| 2-MéthylPentane | % poids | 15% |
| 2-MéthylHexane | % poids | 12% |
| n-Hexane | % poids | 27% |
| Cyclopentane | % poids | 2% |
| Méthylcyclopentane | % poids | 5% |
| Benzène | % poids | 2% |
| Cyclohexane | % poids | 2% |

Pour les deux procédés, la section réactionnelle, i.e. l'unité d'isomérisation **1**, est constituée de deux réacteurs d'isomérisation fonctionnant en série (respectivement réacteur n°1 et réacteur n°2) non représentés sur les figures 1 et 2. Les conditions opératoires pour les deux réacteurs sont présentées ci-après :
Température d'entrée : 120°C ;
Pression d'entrée du réacteur n°1 : 3,5 MPa ;
Pression d'entrée réacteur n°2 : 3,3 MPa ;
Vitesse spatiale : 2,2 h⁻¹ ;
Ratio molaire hydrogène sur hydrocarbure est de 0,1 :1 ;
Teneur en HCI dans le flux d'entrée des réacteurs : 130 parties par millions (ppm) poids.

Le catalyseur utilisé pour les deux procédés dans les deux réacteurs comprend un support en alumine, 7% en poids de chlore par rapport au poids total du catalyseur, et 0,23 % en poids de platine par rapport au poids total du catalyseur. Un appoint en hydrogène est amené à la section réactionnelle **1** via le flux **50** ; un appoint en tétrachlorure de carbone est amené à la section réactionnelle via le flux **51**.

Pour les deux procédés, la charge fraîche disponible est mélangée à 40°C avec le flux de produits recyclés en provenance de la ligne **109**, ces produits étant essentiellement des composés à faible indice d'octane soutiré latéralement du déisohexaniseur **6**. Après séchage, le mélange est séparé en deux flux distincts, le premier flux se dirigeant vers la section réactionnelle via la ligne **101**, et le second flux (appelé aussi ici fluide de lavage) se dirigeant vers la colonne d'adsorption **3** via les lignes **105** et **106** et refroidi à 20°C dans l'échangeur **8**.

Le premier flux, correspondant à une fraction représentant 85% volumique du débit total du mélange, est pompé jusqu'à 3,8 MPa, soit une pression suffisante pour être introduite dans l'unité réactionnelle d'isomérisation **1**. Le fluide de lavage, correspondant à une fraction représentant 15% volumique du débit total du mélange, est envoyé vers la colonne d'absorption **3** après détente à une pression de 1,7 MPa, soit légèrement supérieure à la pression opératoire de cette dernière.
Pour le procédé selon l'invention, la colonne d'absorption **3** est une colonne à garnissage, composé de deux sections. Une première section, appelée ici section inférieure, est comprise entre le fond et l'alimentation en fluide de lavage. Une deuxième section, appelée ici section supérieure, est comprise entre la tête de la colonne et l'alimentation en fluide de lavage. L'effluent liquide issu de la colonne de stabilisation **2** via le ballon de reflux **13** est injecté en tête de la section supérieure de la colonne d'absorption **3**.
Pour les deux procédés, l'effluent de la section réactionnelle d'isomérisation est dirigé, via la ligne **102**, vers la colonne de stabilisation **2**. La teneur en acide chlorhydrique (HCI) dans l'effluent est de 130 parties par millions (ppm) en poids.

La section de séparation **4** des composés à faible indice d'octane consiste, pour les deux procédés, en un déisohexaniseur **6**. Le fond de la colonne de stabilisation **2** est envoyé vers le déisohexaniseur **6**. Le déisohexaniseur **6** se présente sous la forme d'une colonne de séparation dont les produits de tête issus de la ligne **108**. On récupère en fond de colonne de distillation **6** via la ligne **110** un flux enrichi en composés à indice d'octane élevé et en composés C₆₊ naphténiques réfractaires ainsi purgés afin d'éviter leur accumulation. Une coupe intermédiaire, enrichie notamment en n-hexane, est prélevée en soutirage latéral et est recyclée dans la section réactionnelle **1** via la ligne **109**.

Le tableau 2 ci-après décrit les paramètres opératoires de l'ensemble des unités réactionnelles selon l'art antérieur (procédé 1) et selon l'invention (procédé 2). Les plateaux d'alimentation et de soutirage sont repérés par leur numéro d'ordre, l'ensemble des plateaux étant numéroté à partir de un de haut en bas. Le taux de recyclage des hexanes est défini comme le débit du fluide enrichi en n-hexane issus du déisohexaniseur recyclé à la section de réaction d'isomérisation divisé par le débit de la charge en composés hydrocarbonés en mélange avec le flux recyclé, situé en amont de l'unité d'isomérisation.

**Tableau 2: Paramètres opératoires**

| | Procédé 1 (non conforme) | Procédé 2 (invention) |
|---|---|---|
| Colonne de stabilisation **2** | | |
| Nombre de plateaux théoriques | 19 | 19 |
| Pression opératoire (MPa) | 1,8 | 1,6 |
| Plateau d'alimentation | 7 | 7 |
| Puissance requise au rebouilleur (MW) | 5,12 | 5,79 |
| Taux de reflux / Débit charge (pds) | 0,35 | 0,52 |
| Déisohexaniseur **4** | | |
| Nombre de plateaux théoriques | 62 | 62 |
| Pression opératoire (MPa) | 0,29 | 0,29 |
| Plateau d'alimentation | 20 | 20 |
| Plateau de soutirage latéral | 38 | 38 |
| Puissance requise au rebouilleur (MW) | 14,99 | 16,30 |
| Taux de reflux / distillat | 5,05 | 5,25 |
| Taux de recyclage des hexanes | 0,60 | 0,60 |
| Colonne d'absorption **3** de l'unité d'isomérisation **7** | | |
| Nombre de plateaux théoriques | 6 | 10 |
| Pression opératoire (MPa) | 1,6 | 1,4 |
| Plateau d'alimentation charge en composés hydrocarbonés | 1 | 5 |
| Plateau d'alimentation ligne **126** | N/A | 1 |
| Débit de distillats liquides (kg/h) | 1350 | 2100 |
| Unité d'isomérisation **1** | | |
| Débit massique en entrée de réacteur (**101** +**107**) (kg/h) | 63313 | 65210 |
| Débit d'HCl en entrée de réacteur (kg/h) | 8,2 | 8,5 |
| Débit d'appoint en tétrachlorure de carbone (calculé en équivalent HCl) (**51**) (kg/h) | 3,5 | 3,5 |
| Débit de gaz contenant 75% poids d'hydrogène (**50**) (kg/h) | 1009 | 1006 |

Les résultats du procédé 1 correspondent au procédé non conforme à l'invention (figure 1), les résultats du procédé 2 correspondent au procédé selon l'invention (figure 2).
Les compositions des produits issus des flux de la ligne **108** et **110** sont résumées respectivement dans les tableaux 3 et 4 ci-après :

**Tableau 3 : Composition du flux de la ligne 108**

| | | Procédé 1 (non conforme) | Procédé 2 (invention) |
|---|---|---|---|
| Débit Massique | kg / h | 32937 | 33403 |
| i-pentane | % pds | 25 | 25 |
| n-pentane | % pds | 9 | 10 |
| 2,2-dimethylbutane | % pds | 37 | 36 |
| 2,3-dimethylbutane | % pds | 8 | 8 |
| 2-methyl-pentane | % pds | 15 | 15 |
| 2-methyl-hexane | % pds | 3 | 3 |
| cyclopentane | % pds | 1 | 1 |

**Tableau 4 : Composition du flux de la ligne 110**

| | | Procédé 1 (non conforme) | Procédé 2 (invention) |
|---|---|---|---|
| Débit Massique | kg / h | 3350 | 3372 |
| n-hexane | % pds | 5 | 5 |
| methyl-cyclopentane | % pds | 10 | 10 |
| cyclohexane | % pds | 53 | 54 |
| c₇₊ | % pds | 31 | 29 |

En tête de colonne de stabilisation **2**, le flux d'effluent gazeux de la ligne **104** fortement chargé en acide chlorhydrique est injecté dans la colonne d'absorption **3**, dans la section inférieure **A** de celle-ci. Selon l'invention, le distillat liquide issu du ballon de reflux **13** de la colonne de stabilisation **2** est injecté en totalité dans la section supérieure de la colonne d'absorption **3** via la ligne **126**. La composition en composés hydrocarbonés issus du flux de la ligne **126** est résumée dans le tableau 5 ci-après :

**Tableau 5 : Composition en composés hydrocarbonés du flux de la ligne 126**

| | | Procédé 2 (invention) |
|---|---|---|
| Hydrogène | % pds | 0 |
| Méthane | % pds | 0 |
| Ethane | % pds | 3 |
| Propane | % pds | 16 |
| i-Butane | % pds | 61 |
| n-Butane | % pds | 14 |
| i-Pentane | % pds | 5 |
| n-Pentane | % pds | 1 |
| c₆₊ | % pds | 0 |
| HCl | ppm pds | 450 |

Deux flux sont issus de la colonne d'absorption **3**, un flux de fond correspondant essentiellement au fluide de lavage, enrichi en acide chlorhydrique et composés légers ré-entrainés qui est renvoyé vers l'unité d'isomérisation **1** via la ligne **107**, et en tête de l'absorbeur **3** un effluent gazeux appauvri en acide chlorhydrique qui est envoyé vers l'unité de traitement (non représenté sur les figures) via la ligne **111**.

La composition de l'effluent gazeux issu de la colonne d'absorption **3** via la ligne **111** est présentée dans le tableau 6 ci-après :

**Tableau 6 : composition du flux de la ligne 111 (effluent gazeux de la colonne d'absorption 3)**

| | | Procédé 1 (non conforme) | Procédé 2 (invention) |
|---|---|---|---|
| Débit massique total | kg/h | 639 | 1483 |
| Débit HCl | kg/h | 2,8 | 3,5 |
| Hydrogène | % pds | 13 | 6 |
| Méthane | % pds | 29 | 13 |
| Ethane | % pds | 35 | 18 |
| Propane | % pds | 1 | 18 |
| i-Butane | % pds | 1 | 38 |
| n-Butane | % pds | 1 | 6 |
| i-Pentane | % pds | 1 | 1 |
| n-Pentane | % pds | 7 | 0 |
| c₆₊ | % pds | 12 | 0 |
| HCl | ppm pds | 4426 | 2376 |

On observe que la teneur en C₅₊ dans l'effluent gazeux de la colonne d'absorption **3** issu de la ligne **111** est très sensiblement diminuée dans le procédé selon l'invention (exemple 2) par rapport à l'art antérieur (exemple 1).

Le tableau 7 ci-après décrit la composition de l'effluent total envoyé à l'unité de traitement via la ligne **124**, correspondant au flux gazeux de tête issu de la colonne d'absorption **3** via la ligne **111** et à l'effluent liquide vaporisé **123** ce dernier étant nul dans l'exemple pris pour l'invention.

**Tableau 7 : composition du flux de la ligne 124 (effluent vapeur)**

| | | Procédé 1 (non conforme) | Procédé 2 (invention) |
|---|---|---|---|
| Débit Massique | kg/h | 1975 | 1483 |
| Débit HCl | kg/h | 3,5 | 3,5 |
| Hydrogène | % pds | 4 | 6 |
| Méthane | % pds | 9 | 13 |
| Ethane | % pds | 13 | 18 |
| Propane | % pds | 14 | 18 |
| i-Butane | % pds | 30 | 38 |
| n-Butane | % pds | 7 | 6 |
| i-Pentane | % pds | 12 | 1 |
| n-Pentane | % pds | 4 | 0 |
| c₆₊ | % pds | 6 | 0 |
| HCl | ppm pds | 1795 | 2376 |

On observe que le débit d'HCl récupéré dans l'effluent vapeur issue de la ligne **124** est identique dans le procédé selon l'invention et dans le procédé selon l'art antérieur, i.e. 3,5 kg/h. De plus, pour un débit de récupération d'HCl identique pour le procédé 1 (non conforme) et le procédé 2 (conforme à l'invention), le débit massique du flux de la ligne **124** est inférieur dans le procédé 2 que dans le procédé 1, car l'effluent vapeur de la ligne **124** du procédé 2 comprend très peu de composés C₅₊ (seulement 1% pds de i-pentane). Ainsi, à taux de recyclage identique, avec un débit massique diminué dans le cadre du procédé 2, la concentration en HCl est plus importante dans le cadre du procédé 2 que dans le cadre du procédé 1.

Le tableau 8 ci-après décrit le rendement et les propriétés des produits issus du procédé d'isomérisation qui sont constitués par le mélange des flux issus des lignes **108** et **110**. On définit le rendement comme le rapport entre le débit massique de la somme des flux issus des lignes **108** et **110** par le débit de charge la charge en composés hydrocarbonés, éventuellement en mélange avec un flux recyclé, de la ligne **100**, en amont de l'unité d'isomérisation.

**Tableau 8 : Propriétés et rendement des produits issus du procédé d'isomérisation constitués par le mélange des flux issus des lignes 108 et 110**

| | Procédé 1 (non conforme) | Procédé 2 (invention) |
|---|---|---|
| Indice d'octane (RON) | 87,4 | 87,4 |
| Tension de vapeur Reid (MPa) | 0,082 | 0,082 |
| Rendement | 97,4 | 98,7 |

Le procédé selon l'invention permet, pour un produit final obtenu présentant une même tension de vapeur Reid et un même indice d'octane, d'améliorer de manière significative le rendement global la réaction. Pour un taux de récupération de composés chlorés identique le procédé selon l'invention permet d'améliorer le rendement du procédé d'isomérisation par la formation de produits à haut indice d'octane.

## Revendications

1. Procédé d'isomérisation d'une charge de composés hydrocarbonés comportant des composés hydrocarbonés C₅ et/ou C₆, ledit procédé comprenant une boucle de recyclage d'au moins un composé chloré, et dans lequel procédé :
a) on alimente une unité d'isomérisation (1) d'au moins une fraction liquide de ladite charge de composés hydrocarbonés et on effectue l'isomérisation en présence d'un catalyseur chloré ;
b) on alimente une unité de stabilisation (20) comprenant au moins une colonne de stabilisation (2) par l'effluent issu de l'unité d'isomérisation (1) et on effectue une séparation dans ladite unité de stabilisation (2) en :
∘ un effluent gazeux comprenant au moins un composé chloré ;
∘ un effluent liquide comprenant une concentration C_{A} en composés C₅₊ ; et
∘ un flux liquide comprenant au moins un isomérat de la charge de composés hydrocarbonés ;
c) on réalise dans une unité d'absorption (7) comprenant une seule colonne d'absorption (3) :
- un premier contactage entre l'effluent gazeux issu de l'étape b) et un fluide de lavage, l'alimentation dudit effluent gazeux étant effectuée dans la colonne d'absorption (3) en-dessous de l'alimentation dudit fluide de lavage, ledit fluide de lavage étant choisi parmi une fraction liquide complémentaire de ladite charge de composés hydrocarbonés et/ou au moins une partie dudit flux liquide comprenant au moins un isomérat de la charge de composés hydrocarbonés issu de l'étape b); ledit fluide de lavage comprenant une concentration C_{B} en composés C₅₊ avec C_{A} < C_{B} ;
- un second contactage entre le flux gazeux résultant dudit premier contactage et dudit effluent liquide issu de l'étape b) ; l'alimentation dudit effluent liquide étant effectuée en tête de colonne d'absorption (3) en-dessus dudit fluide de lavage ;
d) on extrait de l'unité d'absorption (7) un flux liquide enrichi en composés chlorés et on le renvoie à l'unité d'isomérisation (1) ;
e) on extrait de l'unité de stabilisation (20) ledit flux liquide comprenant au moins un isomérat de la charge de composés hydrocarbonés.

2. Procédé selon la revendication 1, **caractérisé en ce que** le flux liquide comprenant au moins un isomérat de la charge de composés hydrocarbonés de l'étape e) est soumis à une séparation dans une unité de séparation (4) de manière à obtenir au moins un flux présentant un indice d'octane plus élevé que ledit flux liquide de l'étape e) et au moins un flux présentant un indice d'octane plus faible que ledit flux liquide de l'étape e).

3. Procédé selon la revendication 2, **caractérisé en ce que** le flux présentant un indice d'octane plus faible que ledit flux liquide de l'étape e) est au moins en partie recyclé à l'unité d'adsorption (7) en tant que fluide de lavage.

4. Procédé selon les revendications 2 ou 3, **caractérisé en ce que** le flux présentant un indice d'octane plus faible que ledit flux liquide de l'étape e) est au moins en partie recyclé en amont de l'unité d'isomérisation (1), ledit flux recyclé étant mélangé avec la charge de composés hydrocarbonés.

5. Procédé selon l'une quelconque des revendications 1 à 4, **caractérisé en ce que** ladite charge de composés hydrocarbonés, éventuellement en mélange avec ledit flux liquide comprenant au moins un isomérat de la charge de composés hydrocarbonés issu de l'étape b), est séchée en amont de l'unité d'isomérisation (1) et de l'unité d'absorption (7).

6. Procédé selon l'une quelconque des revendications 1 à 5, **caractérisé en ce qu'**on refroidit le fluide de lavage en amont de l'unité d'absorption (7).

7. Procédé selon l'une quelconque des revendications 1 à 6, **caractérisé en ce que** lorsque le fluide de lavage est une fraction liquide complémentaire de ladite charge, le débit de ladite fraction liquide complémentaire de ladite charge est compris entre 5 % et 50 % volumique du débit total de ladite charge.

8. Procédé selon l'une quelconque des revendications 1 à 7, **caractérisé en ce que** la concentration en composés C₄₋ compris dans l'effluent liquide obtenu à l'étape b) est supérieure à 15% en poids.

9. Procédé selon l'une quelconque des revendications 1 à 8, **caractérisé en ce que** le flux gazeux issu du second contactage dans l'unité d'absorption (7) est extrait et traité dans une unité de traitement des effluents gazeux.

10. Procédé selon l'une quelconque des revendications 1 à 9, **caractérisé en ce qu'**une partie de l'effluent liquide issu de l'étape b) est envoyée directement à une unité de traitement des effluents gazeux après vaporisation.

11. Procédé selon l'une quelconque des revendications 1 à 10, **caractérisé en ce que** l'étape a) est réalisée à une température comprise entre 100 et 300°C, à une pression comprise entre 0,2 et 3,5 MPa, avec un rapport molaire hydrogène/hydrocarbures compris entre 0,1:1 et 1:1, et avec une vitesse spatiale comprise entre 0,5 et 10 h⁻¹.

12. Procédé selon l'une quelconque des revendications 1 à 11, caractérisé en que l'étape a) est réalisée en présence d'un catalyseur chloré comprenant un support en alumine et un métal appartenant au groupe VIII.

13. Procédé selon la revendication 12, **caractérisé en ce que** le catalyseur comprend de 2 à 10 % en poids de chlore par rapport au poids total du catalyseur, et de 0,1 à 0,40 % en poids de platine par rapport au poids total du catalyseur.

## Patentansprüche

1. Verfahren zur Isomerisierung einer Charge von Kohlenwasserstoffverbindungen, umfassend C₅- und/oder C₆-Kohlenwasserstoffverbindungen, wobei das Verfahren eine Rückführschleife mindestens einer Chlorverbindung umfasst, und wobei in dem Verfahren:
a) einer Isomerisierungseinheit (1) mindestens eine Flüssigfraktion der Charge von Kohlenwasserstoffverbindungen zugeführt wird, und die Isomerisierung in Anwesenheit eines Chlorkatalysators durchgeführt wird;
b) einer Stabilisierungseinheit (20), umfassend mindestens eine Stabilisierungssäule (2) der Abfluss aus der Isomerisierungseinheit (1) zugeführt wird, und eine Trennung in der Stabilisierungseinheit (2) durchgeführt wird in:
∘ einen gasförmigen Abfluss, umfassend mindestens eine Chlorverbindung;
∘ einen flüssigen Abfluss, umfassend eine Konzentration C_{A} von C₅-Verbindungen; und
∘ einen Flüssigkeitsstrom, umfassend mindestens ein Isomerisat der Charge von Kohlenwasserstoffverbindungen;
c) in einer Absorptionseinheit (7), umfassend eine einzige Absorptionssäule (3), durchgeführt werden:
- ein erstes Inkontaktbringen zwischen dem gasförmigen Abfluss aus Schritt b) und einem Waschfluid, wobei die Zufuhr des gasförmigen Abflusses in der Absorptionssäule (3) unterhalb der Zufuhr des Waschfluids durchgeführt wird, wobei das Waschfluid ausgewählt wird aus einer komplementären Flüssigfraktion der Charge von Kohlenwasserstoffverbindungen und/oder mindestens einem Teil des Flüssigkeitsstroms, umfassend mindestens ein Isomerisat der Charge von Kohlenwasserstoffverbindungen aus Schritt b); wobei das Waschfluid eine Konzentration C_{B} von C₅-Verbindungen umfasst, wobei C_{A} < C_{B};
- ein zweites Inkontaktbringen zwischen dem gasförmigen Strom, der aus dem ersten Inkontaktbringen erhalten wird, und dem flüssigen Abfluss aus Schritt b); wobei die Zufuhr des flüssigen Abflusses am Kopf der Absorptionssäule (3) oberhalb des Waschfluids durchgeführt wird;
d) aus der Absorptionseinheit (7) wird ein mit Chlorverbindungen angereicherter Flüssigkeitsstrom extrahiert, und dieser wird zur Isomerisierungseinheit (1) zurückgeschickt;
e) aus der Stabilisierungseinheit (20) wird der Flüssigkeitsstrom extrahiert, der mindestens ein Isomerisat der Charge von Kohlenwasserstoffverbindungen enthält.

2. Verfahren nach Anspruch 1, **dadurch gekennzeichnet, dass** der Flüssigkeitsstrom, umfassend mindestens ein Isomerisat der Charge von Kohlenwasserstoffverbindungen aus Schritt e), einer Trennung in einer Trenneinheit (4) unterzogen wird, um mindestens einen Strom, der eine höhere Octanzahl aufweist als der Flüssigkeitsstrom aus Schritt e), und mindestens einen Strom, der eine niedrigere Octanzahl aufweist als der Flüssigkeitsstrom aus Schritt e), zu erhalten.

3. Verfahren nach Anspruch 2, **dadurch gekennzeichnet, dass** der Strom, der eine niedrigere Octanzahl aufweist als der Flüssigkeitsstrom aus Schritt e), mindestens teilweise zur Absorptionseinheit (7) als Waschfluid rückgeführt wird.

4. Verfahren nach den Ansprüchen 2 oder 3, **dadurch gekennzeichnet, dass** der Strom, der eine niedrigere Octanzahl aufweist als der Flüssigkeitsstrom aus Schritt e), mindestens teilweise stromaufwärts von der Isomerisierungseinheit (1) rückgeführt wird, wobei der rückgeführte Strom mit der Charge von Kohlenwasserstoffverbindungen gemischt wird.

5. Verfahren nach einem der Ansprüche 1 bis 4, **dadurch gekennzeichnet, dass** die Charge von Kohlenwasserstoffverbindungen, gegebenenfalls in Mischung mit dem Flüssigkeitsstrom, umfassend mindestens ein Isomerisat der Charge von Kohlenwasserstoffverbindungen aus Schritt b), stromaufwärts von der Isomerisierungseinheit (1) und der Absorptionseinheit (7) getrocknet wird.

6. Verfahren nach einem der Ansprüche 1 bis 5, **dadurch gekennzeichnet, dass** das Waschfluid stromaufwärts von der Absorptionseinheit (7) abgekühlt wird.

7. Verfahren nach einem der Ansprüche 1 bis 6, **dadurch gekennzeichnet, dass**, wenn das Waschfluid eine komplementäre Flüssigfraktion der Charge ist, der Durchsatz der komplementären Flüssigfraktion der Charge zwischen 5 Vol.-% und 50 Vol.-% des Gesamtdurchsatzes der Charge beträgt.

8. Verfahren nach einem der Ansprüche 1 bis 7, **dadurch gekennzeichnet, dass** die Konzentration von C₄-Verbindungen, die in dem flüssigen Abfluss enthalten ist, der in Schritt b) erhalten wird, größer als 15 Gew.-% ist.

9. Verfahren nach einem der Ansprüche 1 bis 8, **dadurch gekennzeichnet, dass** der gasförmige Strom aus dem zweiten Inkontaktbringen in der Absorptionseinheit (7) in einer Gasabflussbehandlungseinheit extrahiert und behandelt wird.

10. Verfahren nach einem der Ansprüche 1 bis 9, **dadurch gekennzeichnet, dass** ein Teil des flüssigen Abflusses aus Schritt b) direkt zu einer Gasabflussbehandlungseinheit nach Verdampfung geschickt wird.

11. Verfahren nach einem der Ansprüche 1 bis 10, **dadurch gekennzeichnet, dass** Schritt a) bei einer Temperatur zwischen 100 und 300 °C, bei einem Druck zwischen 0,2 und 3,5 MPa, mit einem Molverhältnis Wasserstoff/Kohlenwasserstoffe zwischen 0,1:1 und 1:1, und mit einer Raumgeschwindigkeit zwischen 0,5 und 10 h⁻¹ durchgeführt wird.

12. Verfahren nach einem der Ansprüche 1 bis 11, **dadurch gekennzeichnet, dass** Schritt a) in Anwesenheit eines Chlorkatalysators durchgeführt wird, der einen Träger aus Aluminium und ein zur Gruppe VIII gehörendes Metall umfasst.

13. Verfahren nach Anspruch 12, **dadurch gekennzeichnet, dass** der Katalysator von 2 bis 10 Gew.-% Chlor, bezogen auf das Gesamtgewicht des Katalysators, und von 0,1 bis 0,40 Gew.-% Platin, bezogen auf das Gesamtgewicht des Katalysators, umfasst.

## Claims

1. A process for the isomerization of a feed of hydrocarbon compounds comprising C₅ and/or C₆ hydrocarbon compounds, said process comprising a loop for recycling at least one chlorinated compound, and in which process:
a) an isomerization unit (1) is supplied with at least one liquid fraction of said feed of hydrocarbon compounds and the isomerization is carried out in the presence of a chlorinated catalyst;
b) a stabilization unit (20) comprising at least one stabilization column (2) is supplied with the effluent obtained from the isomerization unit (1) and a separation is carried out in said stabilization unit (20) into:
• a gaseous effluent comprising at least one chlorinated compound;
• a liquid effluent comprising a concentration C_{A} of C₅₊ compounds; and
• a liquid flow comprising at least one isomerate of the feed of hydrocarbon compounds;
c) the following is carried out in an absorption unit (7) comprising only one absorption column (3):
• a first contact between the gaseous effluent obtained from step b) and a washing fluid, said gaseous effluent being supplied to the absorption column below the supply for said washing fluid, said washing fluid being selected from a complementary liquid fraction of said feed of hydrocarbon compounds and/or at least a portion of said liquid flow comprising at least one isomerate of the feed of hydrocarbon compounds obtained from step b); said washing fluid comprising a concentration C_{B} of C₅₊ compounds, with C_{A} < C_{B};
• a second contact between the gaseous flow resulting from said first contact and said liquid effluent obtained from step b), said liquid effluent being supplied to the head of the absorption column above said washing fluid;
d) a liquid flow enriched in chlorinated compounds is extracted from the absorption unit (7) and returned to the isomerization unit (1);
e) said liquid flow comprising at least one isomerate of the feed of hydrocarbon compounds is extracted from the stabilization unit (20).

2. The process according to claim 1, **characterized in that** the liquid flow comprising at least one isomerate of the feed of hydrocarbon compounds of step e) undergoes a separation in a separation unit (4) in order to obtain at least one flow with a higher octane number than said liquid flow of step e) and at least one flow with a lower octane number than said liquid flow of step e).

3. The process according to claim 2, **characterized in that** at least a portion of the flow with a lower octane number than said liquid flow of step e) is recycled to the absorption unit (7) as washing fluid.

4. The process according to claim 2 or claim 3, **characterized in that** at least a portion of the flow with a lower octane number than said liquid flow of step e) is recycled upstream of the isomerization unit (1), said recycled flow being mixed with the feed of hydrocarbon compounds.

5. The process according to any one of claims 1 to 4, **characterized in that** said feed of hydrocarbon compounds, optionally as a mixture with said liquid flow comprising at least one isomerate of the feed of hydrocarbon compounds obtained from step b), is dried upstream of the isomerization unit (1) and the absorption unit (7).

6. The process according to any one of claims 1 to 5, **characterized in that** the washing fluid is cooled upstream of the absorption unit (7).

7. The process according to any one of claims 1 to 6, **characterized in that** when the washing fluid is a complementary liquid fraction of said feed, the flow rate of said complementary liquid fraction of said feed is preferably in the range 5% to 50% by volume of the total flow of said feed.

8. The process according to any one of claims 1 to 7, **characterized in that** the concentration of C₄- compounds comprised in the liquid effluent obtained in step b) is more than 15% by weight.

9. The process according to any one of claims 1 to 8, **characterized in that** the gaseous flow obtained from the second contact in the absorption unit (7) is extracted and treated in a unit for the treatment of gaseous effluents.

10. The process according to any one of claims 1 to 9, **characterized in that** a portion of the liquid effluent obtained from step b) is sent directly to a unit for the treatment of gaseous effluents after vaporization.

11. The process according to any one of claims 1 to 10, **characterized in that** step a) is carried out at a temperature in the range 100°C to 300°C, at a pressure in the range 0.2 to 3.5 MPa, with a hydrogen/hydrocarbons molar ratio in the range 0.1:1 to 1:1 and with a space velocity in the range 0.5 to 10 h⁻¹.

12. The process according to any one of claims 1 to 11, **characterized in that** step a) is carried out in the presence of a chlorinated catalyst comprising an alumina support and a metal from group VIII.

13. The process according to claim 12, **characterized in that** the catalyst comprises 2% to 10% by weight of chlorine with respect to the total catalyst weight, and 0.1% to 0.40% by weight of platinum with respect to the total catalyst weight.
